# EUROPÄISCHE PATENTSCHRIFT

(11) **EP 1 242 181 B1**
(45) Veröffentlichungstag und Bekanntmachung des Hinweises auf die Patenterteilung: **26.07.2006**
(21) Anmeldenummer: 00990659.5
(22) Anmeldetag: 06.12.2000
(51) Int. Cl.: B01J 23/56, B01J 23/66, C07C 5/08, C07C 5/02

(54) **PD-AG TRÄGERKATALYSATOR ZUR SELEKTIVHYDRIERUNG VON ALKINEN UND DIENEN**
PD-AG SUPPORTED CATALYST FOR THE SELECTIVE HYDROGENATION OF ALKINES AND DIENES
CATALYSEUR SUPPORTE A BASE DE PD ET AG POUR L'HYDROGENATION SELECTIVE D'ALCYNES ET DE DIENES

(30) Priorität: 08.12.1999 DE 19959064
(43) Veröffentlichungstag der Anmeldung: 25.09.2002
(73) Patentinhaber: BASF Aktiengesellschaft, 67056 Ludwigshafen (DE)
(72) Erfinder: FRENZEL, Andrea, 68535 Edingen-Neckarhausen (DE); HESSE, Michael, 67549 Worms (DE); ANSMANN, Andreas, 69168 Wiesloch (DE); SCHWAB, Ekkehard, 67434 Neustadt (DE)
(74) Vertreter: Huhn, Michael
(86) Internationale Anmeldenummer: PCT/EP2000/012283
(87) Internationale Veröffentlichungsnummer: WO 2001/041922

(56) Entgegenhaltungen:
- EP-A- 0 064 301
- EP-A- 0 764 463
- EP-A- 0 839 573
- WO-A-98/37966
- US-A- 5 489 565

## Beschreibung

Die vorliegende Erfindung betrifft das Gebiet der Katalyse. Genauer gesagt, betrifft die vorliegende Erfindung einen neuen Hydrierkatalysator, der es ermöglicht, höher ungesättigte Kohlenwasserstoffe wie Acetylene und Diene in Olefingemischen selektiv zu hydrieren, die nach dem Crackverfahren erhalten wurden. Weitere Gegenstände der vorliegenden Erfindung sind ein Verfahren zur Herstellung eines solchen Katalysators sowie ein Verfahren zur selektiven Hydrierung von Alkinen und Dienen in Olefingemischen mit Hilfe eines solchen Katalysators.

Olefine werden industriell allgemein hergestellt in sogenannten Crackverfahren, beispielsweise dem Steamcracken oder dem katalytischen Cracken per FCC. Dabei werden bestimmte Erdöldestillate auf Temperaturen um die 900°C, bei denen sich aus den vorhandenen Alkanen Olefine bilden, erhitzt. Das erhaltene Rohgemisch wird anschließend destillativ aufgetrennt, wobei die Fraktionen so geschnitten werden, daß die C2 bis C5⁺-Olefine voneinander getrennt werden. Diese erhaltenen Olefine werden dann in die Weiterverarbeitung eingesetzt. Unter den Bedingungen des Crackens bilden sich jedoch auch Alkine (Acetylene) und Diene, wobei deren Anteil von dem Verfahren und den gewählten Versuchsbedingungen abhängt. Diese Alkine und Diene stören jedoch häufig bei der Weiterverarbeitung und Lagerung. Dies liegt zum einen an deren Neigung zur Oligo- und Polymerisation. So können bei der Weiterverarbeitung Produkte gebildet werden, die aus dem Weiterverarbeitungsprodukt häufig aufwendig entfernt werden müssen. Andererseits zeigen die Alkine und Diene eine starke Komplexbildungstendenz. Dies wirkt sich insbesondere dann störend aus, wenn die Olefine im Weiterverarbeitungsschritt einem katalytischen Prozeß unterzogen werden. Die Alkine oder Diene können dann mit dem Katalysator reagieren und diesen desaktivieren oder die Aktivität verändern, was selbstverständlich unerwünscht ist.

Beispielsweise ist es so, daß in dem sogenannten C2-Schnitt, der Ethylen enthält, als unerwünschtes Nebenprodukt Acetylen vorliegt. Ethylen wird in großen Mengen katalytisch zu Polyethylen weiterverarbeitet. Es ist nun so, daß zu einer solchen Polymerisation verwendetes Ethylen im allgemeinen lediglich einen Acetylen-Gehalt von kleiner etwa 1 ppm aufweisen darf. Ähnliches gilt auch für den sogenannten C3-Strom, der neben Propen auch Propadien (Allen) und Propin enthält. Auch Propen wird in großer Menge katalytisch zu Polypropen weiterverarbeitet, in einem Verfahren, das demjenigen des Ethylens gleicht. Ein für die Polymerisation verwendbares Propen darf dabei im allgemeinen lediglich einen Gehalt an Allen und Propin enthalten, der unter etwa 10 ppm ist.

Auch in den anderen Schnitten des Crackverfahrens bilden sich Produkte, die für die Zwecke der Weiterverarbeitung unerwünscht sind. Im sogenannten C4-Schnitt wird abhängig von der Integration in die Wertschöpfungskette Vinylacetylen als Verunreinigung vor der Butadienextraktion hydriert. Wahlweise kann Butadien gezielt in Buten umgewandelt werden, so eine derartige Veredelung des C4-Stroms gewünscht wird. Der sogenannte C5⁺-Schnitt enthält cyclische Pentene und Pentadien, die unter Erhalt der linearen C5-Bausteine und der ungesättigten C5⁺-Komponenten in nicht störende Produkte umgewandelt werden sollten.

Ein Verfahren zum Entfernen der erwähnten Nebenprodukte ist dabei die selektive Hydrierung dieser Alkine und Diene. Dabei werden die Verunreinigungen in der Weiterverarbeitung in nicht störende Komponenten oder vorzugsweise in das Wertprodukt der Kohlenwasserstofffraktion überführt. Das Hauptproblem bei einem solchen Verfahren liegt dabei darin, daß einerseits der Katalysator, der eingesetzt wird, eine ausreichende Aktivität aufweisen muß, um die Nebenprodukte, die ja gegenüber dem Olefin nur in relativ geringen Mengen vorliegen, vollständig zu hydrieren und so den Gehalt an Verunreinigungen auf Werte zu drücken, die bei der Weiterverarbeitung tolerierbar sind. Führt man sich dabei vor Augen, daß teilweise ein Verunreinigungsgehalt von kleiner 1 ppm erreicht werden muß, wie dies bei Polyethylen der Fall ist, so wird klar, daß der in der selektiven Hydrierung eingesetzte Katalysator durchaus eine hohe Aktivität aufweisen muß.

Andererseits muß ein solcher Katalysator auch eine sehr hohe Selektivität oder, anders gesagt, eine geringe spezifische Aktivität gegenüber dem weiter zu verarbeitenden Olefin aufweisen, so daß dieses nicht oder nur in sehr geringem Ausmaß selbst zum entsprechenden Alkan hydriert wird und nicht mehr zur Verfügung steht.

Weiterhin sollten die in den Selektivhydrierungen benutzten Katalysatoren auch die Eigenschaft haben, die Oligomerisierung von Alkinen und Dienen nicht zu katalysieren. Durch diese Reaktion wird nämlich die Bildung von öligen Rückständen bewirkt, die sich auf dem Katalysator akkumulieren. Eine Desaktivierung des Katalysators ist die Folge, die je nach der Menge der gebildeten Nebenprodukte bereits nach weniger als einem Monat eintreten kann.

Nach den im Stand der Technik beschriebenen Verfahren werden zur Selektivhydrierung generell auf Trägern fixierte Katalysatoren von Metallen benutzt, die allgemein in Hydrierungen Anwendung finden, hauptsächlich heterogene Katalysatoren der zehnten Gruppe des Periodensystems, also Ni, Pd, und Pt. In den meisten Fällen wird auf Pd zurückgegriffen.

Der verwendete Träger ist im allgemeinen ein poröses anorganisches Oxid, beispielsweise Kieselerde, Alumosilikat, Titandioxid, Zirkoniumdioxid, Zinkaluminat, Zinktitanat, Spinelle und/oder Mischungen solcher Träger, meist wird jedoch Aluminiumoxid oder auch Siliciumdioxid verwendet. Weiterhin können Promotoren oder andere Zusatzstoffe enthalten sein. Verfahren zur selektiven Hydrierung ungesättigter. Verbindungen in Kohlenwasserstoffströmen, die diese enthalten sind, sowohl als Flüssigphasenhydrierung oder gemischte Gas/Flüssigphasen-hydrierung, in Riesel- oder Sumpffahrweise, wie auch als reine Gasphasen-hydrierung bekannt.

Um die gewünschte Selektivität erreichen zu können, werden die genannten Katalysatoren modifiziert. Es ist allgemein bekannt, daß sich die gewünschten Selektivitätssteigerungen bei den oben genannten Metallen häufig durch die Zugabe von CO während der Hydrierung erreichen lassen. Dieses erfordert jedoch besondere Sicherheitsmaßnahmen, aufgrund der Giftigkeit des CO. Zudem wird so ein COhaltiges Produkt erhalten, das für einige Weiterverwendungen zuerst wieder von diesem gereinigt werden muß.

Im Stand der Technik finden sich eine Vielzahl von Referenzen, die die Verwendung von Palladium-Trägerkatalysatoren, die durch Promotorzusätze modifiziert wurden, in Selektivhydrierungen von Alkinen und Dienen in Kohlenwasserstoffströmen beschreiben. Im Zusammenhang mit der vorliegenden Erfindung sind dabei die nachfolgenden Druckschriften besonders relevant, in denen die Verwendung von Aluminiumoxid als Trägermaterial offenbart ist.

Die EP-A-0 064 301 beschreibt einen Katalysator für die Selektivhydrierung von Acetylen, der aus Pd besteht, das mit Ag modifiziert wurde, als Träger wird α-Aluminiumoxid verwendet. Der Gehalt an Pd beträgt von 0,01 bis 0,025 Gew.%, Ag ist in der 2 bis 10-fachen Menge des Pd vorhanden. In dem fertigen Katalysator ist das Silber über alle Katalysatorteilchen verteilt, während sich 90% des Palladium in einer Randzone von 300 µm befindet.

Die beiden Anmeldungen EP-A-0 686 615 und EP-A-0 780 155 beschreiben einen Katalysator zur selektiven Hydrierung in der Gasphase von Alkinen im C2- oder C3-Strom. Der Katalysator ist Palladium, das mit einem Metall der Gruppe 11 versetzt wurde. Als Trägermaterial wird jeweils Aluminiumoxid verwendet. Mindestens 80% beider Metalle liegen in einer Zone vor, die vom Rand des Katalysatorpartikels bis zu einem Radius reicht, der 80% des Außenradius des Katalysatorpartikels beträgt. Der Palladium-Gehalt beträgt 0,01 bis 0,5 Gew.-% des Katalysators, und das Verhältnis des Metalls der Gruppe 11 zum Palladium beträgt 0,05 bis 0,4 (686 615) bzw. 0,4 bis 3 (780 155). Das bevorzugte Metall der Gruppe 11 ist in beiden Anmeldungen Silber.

Die deutsche Anmeldung mit dem Aktenzeichen 198 39 459.4, angemeldet am 28.08.1998, beschreibt einen Katalysator zur Selektivhydrierung, der mindestens ein hydrieraktives Metall auf einem Aluminiumoxidträger enthält und der ungebraucht im Röntgendiffraktogramm Reflexe zeigt, die den folgenden Netzebenenabständen entsprechen (in 10⁻¹⁰ m): 4,52, 2,85, 2,73, 2,44, 2,31, 2,26, 2,02, 1,91, 1,80, 1,54, 1,51, 1.49, 1,45 und 1,39 mit jeweils bestimmten relativen Intensitäten. Das hydrieraktive Metall ist in einer bevorzugten Ausführungsform Platin und/oder Palladium, das mit Kupfer und/oder Silber dotiert ist.

Die deutsche Anmeldung mit dem Aktenzeichen 198 40 373.9, angemeldet am 3.09.1998 offenbart ein Verfahren, bei dem ungesättigte Verbindungen in Kohlenwasserstoffströmen an einem Katalysator hydriert werden, der mindestens ein Metall der zehnten Gruppe des Periodensystems der Elemente und mindestens ein Metall der elften Gruppe des Periodensystems der Elemente auf einem Aluminiumoxidträger enthält, wobei das Metall oder die Metalle der zehnten Gruppe im wesentlichen in einer oberflächennahen Randschicht des Katalysatorkorns konzentriert ist oder sind, das Metall oder die Metalle der elften Gruppe im wesentlichen gleichmäßig über das Volumen des Katalysatorkorns verteilt vorliegt oder vorliegen, und das Gewichtsverhältnis des Metalls oder der Metalle der elften gruppe zum Metall oder zu den Metallen der zehnten Gruppe höchstens 1,95 beträgt.

Alle oben genannten Veröffentlichungen offenbaren Katalysatoren, bei denen als Träger Aluminiumoxid verwendet wird. Es finden sich nur wenige Referenzen, die Selektivhydrierkatalysatoren offenbaren, die auf einem anderen Träger als Aluminiumoxid angebracht sind.

So findet sich in der DE-A-2 156 544 die Offenbarung eines Verfahrens zur Gewinnung von Ethylen durch selektive katalytische Gasphasenhydrierung von Acetylen im C2-Schnitt der Olefindarstellung, wobei ein Palladiumkatalysator verwendet wird, der auf einem Kieselsäureträger angebracht ist. Der Katalysator ist durch Zink modifiziert.

Diese Verfahren weist jedoch den Nachteil auf, daß die Oligomerbildung in einem für heutige Anforderungen zu hohen Maß eintritt. Weiterhin ist auch die Selektivität häufig nicht ausreichend, und die Zugabe von CO erweist sich als notwendig.

In der EP-A-0 764 463 findet sich die Beschreibung eines Selektivhydrierkatalysators, der Palladium aufweist, das mit einem Promotormetall der Gruppen 1 und 2 des Periodensystems modifiziert wurde. Auch hier ist der Katalysator auf einem Träger auf Basis von Siliciumdioxid angebracht.

Auch hier beobachtet man häufig die Bildung von Oligomeren und somit eine Verminderung der Standzeit des Katalysatorkontakts.

Die DE-P-31 19 850 beschreibt ein Verfahren, das die Selektivhydrierung von Butadien in einer Buten-1-haltigen C4-Fraktion beschreibt. Der verwendete Katalysator ist dabei auf Aluminiumoxid oder auch Siliciumdioxid mit einer sepzifischen Oberfläche von 10 bis 200 m²/g aufgebracht, und der Katalysator besteht aus einer Mischung von Palladium und Silber bzw. Verbindungen dieser Metalle. Der Palladium-Gehalt beträgt 0,05 bis 5 Gew.-%, der Silbergehalt 0,05 bis 1 Gew.-%. Der in dieser Referenz beschriebene Katalysator ist jedoch nur für die Selektivhydrierung von Butadien in C4-Strömen geeignet.

Die deutsche Anmeldung mit dem Aktenzeichen 198 40 372.0, angemeldet am 3.09.1998, lehrt einen Katalysator, der in seiner Aktivmasse 0,05 bis 1,0 Gew.-% mindestens eines Metalls oder einer Verbindung eines Metalls der zehnten Gruppe des Periodensystems der Elemente und 0,05 bis 1,0 Gew.-% mindestens eines Metalls oder einer Verbindung eines Metalls der elften Gruppe des Periodensystems der Elemente enthält, wobei das Gewichtsverhältnis des enthaltenen Metalls der elften Gruppe zum enthaltenen Metall der zehnten Gruppe von 0,95 bis 1.05 beträgt, und der als Träger einen Siliciumdioxid enthaltenen Katalysatorträger mit einer BET-Oberfläche zwischen 2 und 400 m²/g enthält, und wobei mindestens 20 % des Gesamtporenvolumens des Katalysators in Poren mit einem Durchmesser oberhalb von 100 Nanometern vorliegt. Der Katalysator wird zur Entfernung von Alkine, Dienen, und/oder einfach ungesättigten Kohlenwasserstoffen aus Stoffströmen verwendet.

Es kann gesagt werden, dass es bis jetzt nicht möglich war, einen Selektivhydrierkatalysator für Alkine und Diene zu entwickeln, der auf einem anderen Trägermaterial als Aluminiumoxid, beispielsweise Siliciumdioxid, angebracht ist und die gleiche Leistungsfähigkeit aufweist wie auf Aluminiumoxid als Trägermaterial angebrachte Katalysatoren.

Die Aufgabe der vorliegenden Erfindung ist daher die Bereitstellung eines Katalysators zur selektiven Hydrierung von Alkinen und Dienen in C2-C5⁺-Olefingemischen, der auf einem anderen Träger als Aluminiumoxid angebracht ist, wobei der Katalysator jedoch die gleiche Leistungsfähigkeit aufweisen soll wie solche, bei denen Aluminiumoxid als Träger verwendet wurde. Weiterhin soll ein solcher Katalysator möglichst einfach herzustellen sein. Vorzugsweise soll der Katalysator auch leichter sein als solche, bei denen Aluminiumoxid als Trägermaterial verwendet wurde.

Diese Aufgabe wird gelöst durch einen Katalysator zum selektiven Hydrieren von Alkinen und Dienen in C2-C5⁺-Olefingemischen, wobei der Katalysator enthält
(a) Palladium,
(b) ein Metall der elften Gruppe des Periodensystems,
(c) gegebenenfalls eine Verbindung eines Metalls der ersten oder zweiten Gruppe des Periodensystems,
und diese Metalle auf einem Träger aus Siliciumdioxid aufgebracht sind, und wobei das Metall der elften Gruppe homogen über den Querschnitt des Katalysatorkorns verteilt ist und sich mehr als 80 Gew.-% des Palladiums in einer Schicht von nicht mehr als 0,6 mm Dicke, die von der geometrischen Oberfläche des Katalysatorpartikels begrenzt wird, befindet.

Diese Aufgabe wird weiterhin gelöst durch ein Verfahren zur Herstellung eines derartigen Katalysators, wobei dieses Verfahren dadurch gekennzeichnet ist, dass zuerst auf dem Träger das Metall der elften Gruppe homogen aufgebracht wird und anschließend Palladium aufgebracht wird. Vorzugsweise wird das Metall der elften Gruppe vor der Verformung des Trägers eingearbeitet, das Aufbringen von Palladium geschieht vorzugsweise durch Tränken mit einer Lösung eines Salzes des Palladiums.

Ein derartiger Katalysator läßt sich vorteilhafterweise in Selektivhydrierungen von Alkinen und Dienen in C2-C5⁺-Olefingemischen verwenden. Unter Olefingemischen werden dabei im Zusammenhang mit der vorliegenden Erfindung vorzugsweise sogenannte Kohlenwasserstoffströme verstanden, also die beim Cracken von Erdöldestillaten oder Erdgas erhaltenen Produkte, die zum Großteil Olefine enthalten. Das erfindungsgemäße Verfahren kann aber auch zur Selektivhydrierung von Alkinen und Dienen in Olefingemischen benutzt werden, die in anderen, einem Fachmann bekannten Verfahren erhalten wurden.

Es wurde überraschend gefunden, dass durch das selektive Aufbringen des Metalls der elften Gruppe und Palladium ein selektiver Hydrierkatalysator für Alkine und Diene zugänglich ist, der auf einem Trägermaterial angebracht ist, das nicht Aluminiumoxid ist. Der erhaltene Katalysator ist jedoch ebenso leistungsfähig wie solche, die auf einem Aluminiumoxid-Träger angebracht sind.

Als Trägermaterial dient im Rahmen der vorliegenden Erfindung Siliciumdioxid. Siliciumdioxid weist den Vorteil auf, spezifisch wesentlich leichter zu sein als Aluminiumoxid. Trägerkatalysatoren mit geringem Schüttgewicht sind generell kostengünstiger als Katalysatoren mit hohem Schüttgewicht.

Das in der vorliegenden Erfindung benutzte Trägermaterial weist eine BET-Oberfläche von 20 bis 400 m²/g, vorzugsweise 100 bis 160 m²/g, und ein Porenvolumen von 0,1 bis 1,5 ml/g, vorzugsweise 0,7 bis 1,2 ml/g, auf.

Palladium ist in Mengen von 0,005 bis 1 Gew.-% vorzugsweise 0,02 bis 0,6 Gew.-% bezogen auf die Gesamtmasse, in dem erfindungsgemäßen Katalysator vorhanden.

Palladium ist in einer oberflächennahen Randschicht des Trägers mit mehr als 80 Gew.-%, vorzugsweise mehr als 90 Gew.-% und in besonders bevorzugter Weise mehr als 95 Gew.-% in einer Schicht von nicht mehr als 0,6 mm Dicke, die von der geometrischen Oberfläche des Katalysatorpartikels begrenzt wird, enthalten. Vorzugsweise ist diese Schicht nicht dicker als 0,45 mm.

Ein wichtiges Element des Katalysators nach der vorliegenden Erfindung ist das Promotormetall, das ein Metall aus der elften Gruppe des Periodensystems, also Kupfer, Silber, oder Gold ist. Die Zugabe dieses Metalls sowie dessen spezifische Anordnung in dem Hydrierkatalysator nach der vorliegenden Erfindung erlauben die selektive Hydrierung von Alkinen und Dienen mit einer hohen Aktivität und Selektivität. Gleichzeitig wird die Neigung zur Bildung von Oligomeren und somit die daraus resultierende Katalysatordesaktivierung herabgesetzt.

Das Metall der elften Gruppe liegt in Mengen von 0,005 bis 1 Gew.-%, vorzugsweise 0,05 bis 0,6 Gew.-% bezogen auf die Gesamtmmasse, in den erfindungsgemäßen Katalysator vor. Das Verhältnis von Metall der elften Gruppe zu Palladium liegt im Bereich von 0,01 bis 100 bezogen auf Palladium. Bevorzugterweise liegt dieses Verhältnis in einem Bereich von 0,5 bis 30, meistbevorzugt 1,5 bis 20, in dem die besten Resultate erzielt werden konnten. Es ist weiterhin bevorzugt, wenn das Metall der elften Gruppe Silber ist.

Der Katalysator nach der vorliegenden Erfindung kann so aufgebaut sein, dass jeweils nur ein Metall der elften Gruppe und Palladium bzw. Verbindungen dieser Metalle vorhanden sind. Es können jedoch auch zwei oder mehr Metalle der elften Gruppe bzw. deren Verbindungen unabhängig voneinander in dem Katalysator vorliegen.

Es ist besonders bevorzugt, wenn der Katalysator nach der vorliegenden Erfindung Palladium und Silber enthält.

Das Metall der elften Gruppe befindet sich in dem erfindungsgemäßen Katalysator über den gesamten Querschnitt des Katalysatorkorns verteilt.

Dies kann nach dem Fachmann bekannten Methoden wie der sog. "incipient wetness"-Methode durch Auftränkung auf den verformten Träger erreicht werden. Bevorzugterweise wird das Metall der elften Gruppe vor der Verformung des Trägers aufgebracht. Auch hierzu können solche dem Fachmann bekannte Methoden wie
- Tränkung des gefällten Trägermaterials mit dem Metall der elften Gruppe oder einer Verbindung desselben,
- Kofällung der Trägerverbindung und des Metalls der elften Gruppe oder einer Verbindung desselben,
- Vermischung der Trägermaterials mit dem Metall der elften Gruppe oder einer Verbindung desselben im trockenen oder feuchten Zustand,
- Aufdampfen des Metalls der elften Gruppe auf das Trägermaterial verwendet werden.

So wird zunächst ein Träger erhalten, der das Metall der elften Gruppe homogen über den Trägerquerschnitt verteilt enthält.

In jedem Fall geschieht das Aufbringen des Metalls der elften Gruppe in einem Schritt, der vor dem Aufbringen des Palladiums erfolgt.

Das Palladium läßt sich ebenfalls mit den üblichen, einem Fachmann bekannten Maßnahmen, auf dem Träger fixieren. Auch hier ist es jedoch wiederrum bevorzugt, wenn dieses Aufbringen durch Tränken mit einer Lösung eines geeigneten Salzes des Palladiums erfolgt. Vorzugsweise wird dieses so durchgeführt, dass die Lösung praktisch vollständig vom Porenvolumen des Trägers aufgenommen wird ("incipient wetness"-Methode). Dabei muß das Aufnahmevermögen des Trägers für die Tränklösung jedoch nicht voll ausgeschöpft werden, die Tränklösung kann also in einer Menge von weniger als 100%, beispielsweise nicht mehr als 95 Vol.-%, nicht mehr als 90 Vol.-%, oder nicht mehr als 85 Vol.-% des vom zu tränkenden Träger aufnehmbaren Flüssigkeitsvolumen verwendet werden. Die Konzentration der Salze in der Lösung wird so bemessen, dass nach Tränkung und Umwandlung des getränkten Trägers zum fertigen Katalysator die abzuscheidenden Komponenten in der gewünschten Konzentration auf dem Katalysator vorliegen. Die Salze werden so gewählt, dass sie keine bei der Katalysatorherstellung oder dessen späterer Verwendung störenden Rückstände hinterlassen. Meistens werden Nitrate oder Ammoniumsalze verwendet.

Es hat sich gezeigt, dass durch das zunächst erfolgende, homogene Aufbringen des Metalls der elften Gruppe die erfindungsgemäße gewünschte Verteilung des Palladiums erreicht wird.

Die erfindungsgemäßen Katalysatoren können noch weitere Promotormetalle enthalten, die aus der ersten und zweiten Gruppe des Periodensystems ausgewählt sind. Vorzugsweise werden benutzt Natrium, Kalium, Calcium oder Barium. Das Aufbringen erfolgt durch einen Fachmann bekannte, geeignete Methoden, beispielsweise durch Tränken, und zwar parallel zur Aufbringung des Palladiums und des Metalls der elften Gruppe und unabhängig von der gewählten Reihenfolge der Aufbringung.

Nach dem Aufbringen der Metalle werden die erhaltenen Rohkatalysatoren bei den üblichen Temperaturen getrocknet und kalziniert, wobei dies in einem einzigen Schritt oder zwei voneinander getrennten Schritten durchgeführt werden kann. Das Trocknen erfolgt bei Temperaturen von 50°C bis 250°C, vorzugsweise 70°C bis 100°C. Das Calcinieren wird bei Temperaturen von 250°C bis 700°C, vorzugsweise 300°C bis 650°C durchgeführt, wobei beispielsweise Drehrohre, Bandcalcinierer oder Muffelöfen verwendet werden können. Die Körper haben die übliche Form, etwa Stränge, Kugeln, Ringe, Tabletten, und werden durch beispielsweise Tablettieren oder Extrudieren der Träger dargestellt.

Die erfindungsgemäßen Katalysatoren eignen sich zum selektiven Hydrieren von generell allen Alkinen und Dienen mit einer Kohlenstoffzahl von C2 bis C5 in Gemischen von diesen mit Olefinen, generell in beim Cracken erhaltenen Kohlenwasserstoff-Strömen. Das Hydrieren kann in der Gasphase und in der Flüssigphase erfolgen, analog zu bekannten, heterogenen katalytischen Hydrierverfahren. Das Hydrieren kann als reines Gasphasenverfahren als auch als Gas/Flüssigphasenverfahren durchgeführt werden. Diese Verfahren sind einem Fachmann bekannt. Die Reaktionsparameter, beispielsweise Kohlenwasserstoffdurchsatz, Temperatur und Druck werden analog zu denen bekannter Verfahren gewählt.

Die eingesetzte Wasserstoffmenge beträgt das 0,8 bis 5-fache, vorzugsweise das 0,95 - 2-fache der stöchiometrisch zum vollständigen Umsatz nötigen Menge.

Beispiele für Hydrierverfahren, bei denen der erfindungsgemäße Katalysator verwendet werden kann, sind nachfolgend aufgeführt
- selektive Hydrierung von Acetylen in C2-Strömen zu Ethylen (nachfolgend, als "Verfahren A" bezeichnet)
- selektive Hydrierung von Propin und/oder Propadien in C3-Strömen zu Propylen ("Verfahren B")
- selektive Hydrierung von 1-Butin, 2-Butin, 1,2-Butadien und/oder Vinylacetylen in C4-Strömen zu 1,3-Butadien, 1-Buten, cis- und/oder trans-2-Buten ("Verfahren C")
- selektive Hydrierung von 1-Butin, 2-Butin, 1,2-Butadien, 1,3-Butadien und/oder Vinylacetylen in C4-Strömen zu 1-Buten, cis- und/oder trans-2-Buten, bei butadienreichen C4-Strömen ("Roh-C4-Schnitt") oder butadienarmen C4-Strömen (Raffinat I") ("Verfahren D)
- selektive Hydrierung ungesättigter Verbindungen und/oder ungesättigter Substituenten aromatischer Verbindungen in C5⁺-Strömen zu höher gesättigten Verbindungen und/oder aromatischen Verbindungen mit höher gesättigten Substituenten ("Verfaluen E")

Verfahren A wird üblicherweise als ein- oder mehrstufiges Gasphasenverfahren mit einer Raumgeschwindigkeit des gasförmigen C2-Stroms von 500 m³/m^{3*}h, bezogen auf das Katalysatorvolumen, bis 10 000 m³/m^{3*}h bei einer Temperatur von 0°C bis 250°C und einem Druck von 0,01 bar bis 50 bar durchgeführt.
Verfahren B wird üblicherweise als ein- oder mehrstufiges Gasphasenverfahren mit einer Raumgeschwindigkeit des gasförmigen C3-Stroms von 500 m³/m³*h, bezogen auf das Katalysatorvolumen, bis 10 000 m³/m³*h bzw. Gas-/Flüssig-phasenverfahren mit einer Raumgeschwindigkeit des flüssigen C3-Stroms von 1 m³/m³*h, bezogen auf das latalysatorvolumen, bis 50 m³/m³*h bei einer Temperatur von 0°C bis 180°C und einem Druck von 0,01 bar bis 50 bar durchgeführt.

Verfahren C wird üblicherweise als Gas-/Flüssigphasenverfahren mit einer Raumgeschwindigkeit des flüssigen C4-Stroms von 1 m³/m³*h, bezogen auf das Katalysatorvolumen, bis 50 m³/m³*h bei einer Temperatur von 0 °C bis 180°C und einem Druck von 2 bar bis 50 bar durchgeführt. Verfahren C kann beispielsweise als selektive sogenannte "front end-Vinylacetylenhydrierung" vor einer Butadienextraktion eingesetzt werden.

Verfahren D wird üblicherweise als ein- oder zweistufiges Gas-/Flüssigphasenverfahren mit einer Raumgeschwindigkeit des flüssigen C4-Stroms im Bereich von 0,1 m³/m³*h, bezogen auf das Katalysatorvolumen, bis 60 m³/m^{3*}h, vorzugsweise von 1 m³/m^{3*}h bis 50 m³/m^{3*}h, bei einer Reaktor-eingangstemperatur im Bereich von 20°C bis 90°C, vorzugsweise von 20°C bis 70°C, und einem Druck im Bereich von 5 bar bis 50 bar, vorzugsweise von 10 bar bis 30 bar durchgeführt. Beispielsweise wird das Verfahren zweistufig durchgeführt, wobei der Butadiengehalt, der in typischen C4-Strömen aus Steamcrackem im Bereich von 20 Gew.-% bis 80 Gew.-%, bezogen auf den Gesamtstrom, liegt, in der ersten Stufe bis auf einen Gehalt im Bereich von 0,1 Gew.-% bis 20 Gew.-% und in der zweiten Stufe bis auf den gewünschten Restgehalt im Bereich von wenigen Gew.-ppm bis zu etwa 1 Gew.-% verringert wird. Es ist ebenso möglich, die Gesamtreaktion auf mehr als zwei Reaktoren, beispielsweise drei oder vier, zu verteilen. Die einzelnen Reaktionsstufen können unter teilweiser Rückführung des Kohlenwasserstoffstroms betrieben werden, das Rücklaufverhältnis liegt üblicherweise im Bereich von 0 bis 30. Isobuten bleibt bei der Durchführung von Verfahren D im wesentlichen unverändert erhalten und kann vor oder nach der Durchführung von Verfahren D mit bekannten Methoden aus dem C4-Strom abgetrennt werden. Verfahren D kann beispielsweise als Butadienhydrierung im C4-Strom (wenn Butadien nicht als Wertprodukt gewonnen werden soll) oder als selektive sogenannten "tail end-Vinylacetylenhydrierung" nach der Butadienextraktion aus dem C4-Strom verwendet werden.

Verfahren E wird vorzugsweise als Gas-/Flüssigphasenverfahren mit einer Raumgeschwindigkeit des flüssigen C5+-Stroms von 0,5 m³/m³*h, bezogen auf das Katalysatorvolumen, bis 30 m³/m³*h bei einer Temperatur von 0°C bis 180°C und einem Druck von 2 bar bis 50 bar durchgeführt. Verfahren E kann beispielsweise als selektive Pyrolysebenzinhydrierung, als selektive Hydrierung von Olefinen in Reformatströmen oder Koksöfen-Kondensaten, zur Hydrierung von Styrol zu Ethylbenzol eingesetzt werden.

Durch das Zufügen von Metallen der elften Gruppe wird in den erfindungsgemäßen Katalysatoren der Träger derart vorkonditioniert, dass die Bildung von Oligomeren während der Hydrierung deutlich gesenkt wird, im Gegensatz zu sonstigen, auf den gleichen Trägern angebrachten Hydrierkatalysatoren. Die Standzeit des Kontaktes erhöht sich somit deutlich. Weiterhin ist auch die häufig immer noch benötigte Zugabe von CO als selektivitätssteuerndes Mittel nicht mehr notwendig. In den erfindungsgemäßen Katalysatoren kann das hydrieraktive Palladium selbst mit hohen Überschüssen an Promotormetall der elften Gruppe versehen werden, ohne dass ein Aktivitätsverlust beim Hydrieren beobachtet wird.

Die vorliegende Erfindung wird nun in den nachfolgenden Beispiele erläutert.

### Beispiel 1 (erfindungsgemäßer Katalysator A)

Ein erfindungsgemäßer Katalysator wurde hergestellt, indem ein Siliciumdioxid-Träger (4 mm-Extrudate, BET-Oberfläche zwischen 120 und 140 m²/g, Porenvolumen zwischen 0,8 und 0,95 ml/g) in Strangform derart hergestellt wurde, dass während des Kollerschrittes Silber in Form von Silbernitrat mit bezogen auf die eingesetzte SiO₂₋Menge 0,05 Gew.-% zur Kollermasse zugegeben wurde. Nach der Extrusion zu 4 mm-Strängen wurde der Träger calciniert und anschließend mit bezogen auf die eingesetzte Trägermasse 0,025 Gew.-% Palladium in Form von Palladiumnitrat bei Raumtemperatur imprägniert. Dabei wurden als Lösungsvolumen 90% der Wasseraufnahme des Trägers eingesetzt. Der Kontakt wurde bei 80°C getrocknet und anschließend bei 500°C calciniert.

### Beispiel 2 (erfindungsgemäßer Katalysator B)

Ein erfindungsgemäßer Katalysator wurde hergestellt, indem ein Siliciumdioxid-Träger (4 mm-Extrudate, BET-Oberfläche zwischen 120 und 140 m²/g, Porenvolumen zwischen 0,8 und 0,95 ml/g) in Strangform derart hergestellt wurde, daß während des Kollerschrittes Silber in Form von Silbernitrat mit bezogen auf die eingesetzte SiO₂₋Menge 0,2 Gew.-% zur Kollermasse zugegeben wurde. Nach der Extrusion zu 4 mm-Strängen wurde der Träger calciniert und anschließend mit bezogen auf die eingesetzte Trägermasse 0,06 Gew.-% Palladium in Form von Palladiumnitrat bei Raumtemperatur imprägniert. Dabei wurden als Lösungsvolumen 90% der Wasseraufnahme des Trägers eingesetzt. Der Kontakt wurde bei 80°C getrocknet und anschließend bei 500°C calciniert.

### Vergleichsbeispiel 1 (Vergleichskatalysator C)

Ein Vergleichskatalysator wurde hergestellt, indem ein Aluminiumoxid-Träger in Strangform mit einer BET-Oberfläche von 8m²/g mit einer salpetersauren, wässrigen Lösung von bezogen auf die eingesetzte Trägermasse 0,045 Gew.-% Silber in Form von Silbernitrat und von bezogen auf die eingesetzte Trägermasse 0,025 Gew.-% Palladium in Form von Palladiumnitrat bei Raumtemperatur imprägniert wurde (integrales Verhältnis von Ag/Pd = 6,7:1). Dabei wurden als Lösungsvolumen 90% der Wasseraufnahme des Trägers eingesetzt. Der Kontakt wurde bei 80°C getrocknet und anschließend bei 400°C calciniert.

### Vergleichsbeispiel 2 (Vergleichskatalysator D)

Ein weiterer Vergleichskatalysator wurde entsprechend Beispiel 8 der Anmeldung DE 2156544 hergestellt, wobei ein 0,025 % Palladium und 0,01 % Zink enthaltender Katalysator auf einem Kieselsäuregel-Träger in Form eines 4 mm Strangextrudats erhalten wurde.

### Beispiel 3

### Performancetest der Kontakte A bis D

Die Katalysatoren A bis D wurden in der Selektivhydrierung von Acetylen in einem C2-Strom enthaltend ca. 1 % Alkin bei 20 bar und einer Belastung von 3000 1/h und einem 80%igen H₂-Überschuß bezogen auf die stöchiometrische Menge getestet. Die jeweilige Aktivität, gemessen an der Reaktionstemperatur, Stabilität, gemessen an der Desaktivierungsräte des Umsatzes, und Selektivität, gemessen an der Neigung zur Oligomerenbildung, wurden geprüft und verglichen. Tabelle 1 gibt die Testergebnisse der Kontakte A bis D wider.

**Tabelle 1**

| **Katalysator** | **A** | **B** | **C** | **D** |
|---|---|---|---|---|
| Rüttelgewicht | 470 | 470 | 1100 | 470 |
| Palladium [Gew.-%] | 0,025 | 0,06 | 0,025 | 0,025 |
| Ag/Pd-Verhältnis | 2 | 3,3 | 1,8 | |
| CO-Zugabe zur Performancestabilisierung | nicht benötigt | nicht benötigt | nicht benötigt | benötigt |
| Reaktionstemperatur [°C] | 65 | 50 | 45 | 65 |
| Deaktivierungsrate des Umsatzes [%/h] | -0,048 | -0,002 | -0,001 | -0,159 |
| Oligomerenbildung [g/h] | 0,024 | 0,012 | 0,014 | 0,051 |

Die enthaltenen Ergebnisse zeigen, daß die Zugabe von Silber zum Träger hinsichtlich aller Kenngrößen verbesserte Katalysatoren liefert (A und B vs. D). Weiterhin wird deutlich, daß die Zugabe von Silber zu den Trägern die Zugabe von CO zur Selektivitätserhöhung unnötig macht (A und B vs. D), und daß höhere Ag/Pd-Verhältnisse zu geringerer Koksbildung und langsamerer Deaktivierungsrate führen (A vs.B). Zudem läßt sich durch Anheben der Palladiummenge eine deutliche Aktivitätssteigerung erzielen (A vs. B und D).

Mit dem Katalysator A und insbesondere dem Katalysator B lassen sich im Vergleich zum Kontakt C deutlich leichterere und somit für den Betreiber kostengünstigere Katalysatoren erhalten, die sich durch eine dem Stand der Technik (Kontakt C) entsprechende, sehr gute Performance auszeichnen.

### Beispiel 4

Die erfindungsgemäßen Katalysatoren E bis U wurden gemäß der nachfolgenden Vorschrift und unter Variation der in der Tabelle 2 angegebenen Veränderungen einzelner Herstellparameter erhalten:

Ein mit Silber dotierter SiO₂-Träger in Form von 4 mm Extrudaten wurde hergestellt, indem aus einer ammoniakalischen Natrium-Wasserglas-Lösung durch Zugabe von Schwefelsäure Kieselsäure bei pH 6 gefällt wurde, welche von der wässrigen Phase abfiltriert, von Leitfähigkeit durch Waschen mit Wasser befreit und zu Pulver (herstellbedingt schwankendes Rüttelgewicht zwischen 350 und 450 g/l, Wassergehalt: ca. 25 %) versprüht wurde. Das erhaltene Sprühpulver wurde mit Wasser und unter Zugabe einer definierten Menge Silber, bezogen auf die eingesetzte Feststoffmasse, in Form von wässriger Silbernitratlösung eine Stunde lang zu einer extrudierbaren Masse verknetet (sog. Kollern). Die gekollerte Masse wurde unter Erhalt von 4 mm-Extrudaten zu Strängen verformt. Die Stränge wurden bei 120 °C getrocknet und bei definierter Temperatur calciniert. Erhalten wurde ein Produkt mit einer BET-Oberfläche von 110 bis 160 m²/g und einem Rüttelgewicht zwischen 440 und 480 g/l. Die genauen Oberflächenwerte und Rüttelgewichte sind in Tabelle 1 angegeben.

Dieses Trägermaterial wurde anschließend mit einer bezogen auf die eingesetzte Trägermasse definierten Menge Palladium in Form von wässriger Palladiurnnitrat-Lösung bei Raumtemperatur bewegt getränkt. Dabei wurden als Lösungsvolumen 95 % der Wasseraufnahme des Trägers eingesetzt. Das erhaltene Material wurde unter Luftzugabe bei definierter Temperatur bewegt getrocknet und anschließend über 1 Stunde bei definierter Temperatur bewegt calciniert. Die genauen Herstellparameter sind Tabelle 1 zu entnehmen.

### Performancetests der Kontakte E bis U

Die Katalysatoren E bis U wurden bei 1 bar im geraden Durchgang getestet. Die GHSV betrug 3000 1/h. Der Strom setzte sich aus 1 Vol.-% Acetylen in Ethylen zusammen und wurde mit 1,8 Äquivalenten H₂ bezogen auf die eingesetzte C₂H₂-Menge versetzt. Die für einen 90%igen Umsatz des Acetylens notwendigen Temperaturen sowie die erhaltenen Selektivitätswerte sind aus Tabelle 2 ersichtlich.

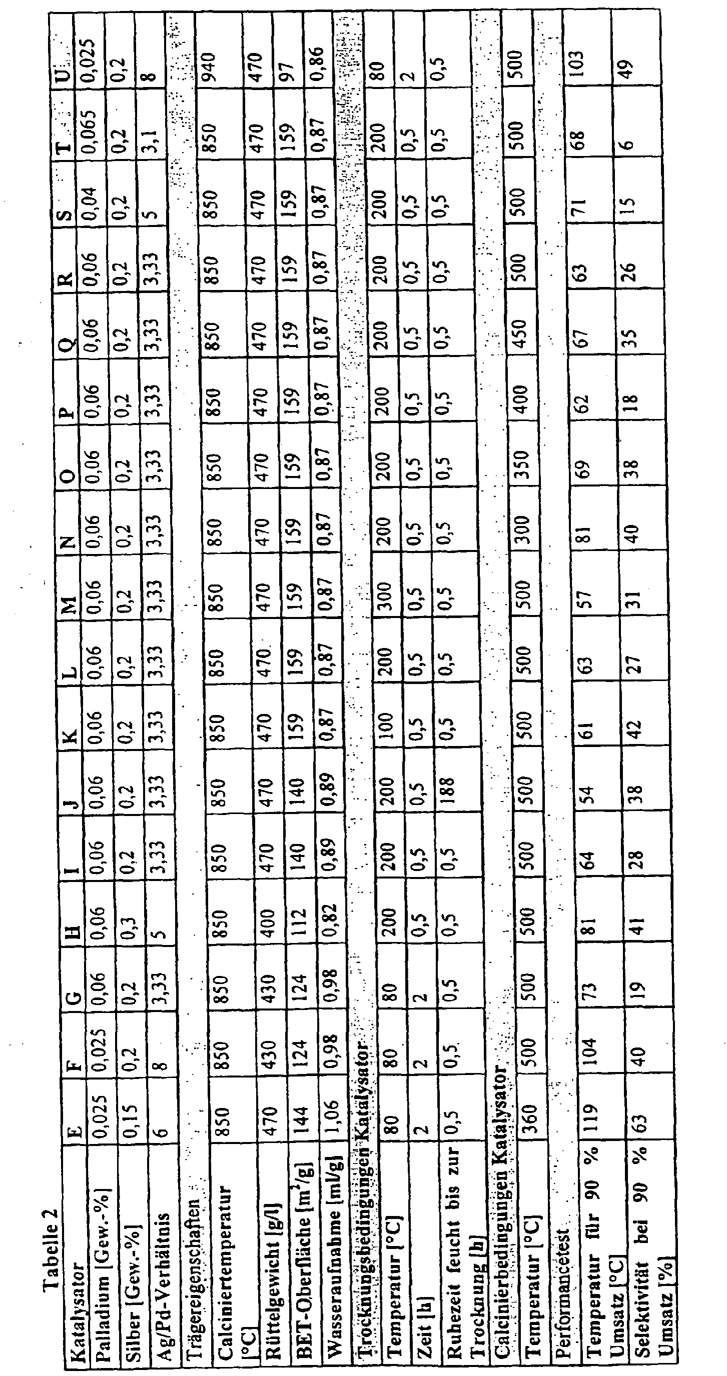

## Patentansprüche

1. Katalysator zum selektiven Hydrieren von Alkinen und Dienen in C2-C5⁺⁻Olefingemischen, wobei diese Katalysatoren enthalten
(a) Palladium
(b) ein Metall der elften Gruppe des Periodensystems
(c) gegebenenfalls eine Verbindung eines Metalls der ersten oder zweiten Gruppe des Periodensystems,
diese Metalle auf einem Träger aus Siliciumdioxid angebracht sind und wobei das Metall der elften Gruppe homogen über den Querschnitt des Katalysatorkorns verteilt ist und sich mehr als 80 Gew.-% des Palladiums in einer Schicht von nicht mehr als 0,6 mm Dicke, die von der geometrischen Oberfläche des Katalysatorpartikels begrenzt wird, befindet.

2. Katalysator nach Anspruch 1, **dadurch gekennzeichnet, dass** das Metall der elften Gruppe Silber ist.

3. Katalysator nach Anspruch 1 oder 2, **dadurch gekennzeichnet, dass** Palladium in einer Menge von 0,005 bis 1 Gew.-%, vorzugsweise 0,02 bis 0,6 Gew.-%, bezogen auf die Gesamtmasse vorhanden ist.

4. Katalysator nach einem Ansprüche 1 bis 3, **dadurch gekennzeichnet, dass** das Metall der elften Gruppe in einer Menge von 0,005 bis 1 Gew.-%, vorzugsweise 0,05 bis 0,6 Gew.-%, vorhanden ist und das Verhältnis von Metall der elften Gruppe zu Palladium von 0,01 bis 100, vorzugsweise von 0,5 bis 30, meistbevorzugt 1,5 bis 20, reicht.

5. Verfahren zur Herstellung eines Katalysators nach einem der Ansprüche 1 bis 4, **dadurch gekennzeichnet, dass** zuerst auf dem Träger das Metall der elften Gruppe derart aufgebracht wird, dass es homogen verteilt ist und anschließend Palladium aufgebracht wird.

6. Verfahren nach Anspruch 5, **dadurch gekennzeichnet, dass** das Aufbringen des Metalls der elften Gruppe vor oder nach der Verformung des Trägers, vorzugsweise vor der Verformung des Trägers erfolgt.

7. Verfahren nach Anspruch 6, **dadurch gekennzeichnet, dass** das Metall der elften Gruppe durch Einkneten in die Trägermasse während des Kollerschritts eingebracht wird.

8. Verfahren zur selektiven Hydrierung von Alkinen und Dienen in C2-C5⁺⁻Olefingemischen, vorzugsweise in C2- oder C3-Strömen der Olefinherstellung durch Cracken, **dadurch gekennzeichnet, dass** ein Katalysator nach einem der Ansprüche 1 bis 4 in der Hydrierung verwendet wird.

9. Verfahren nach Anspruch 8, **dadurch gekennzeichnet, dass** die Hydrierung bei Temperaturen von 0 bis 250°C und Drücken von 0,01 bis 50 bar durchgeführt wird.

## Claims

1. A catalyst for the selective hydrogenation of alkynes and dienes in C2-C5⁺-olefin mixtures, the catalyst comprising
(a) palladium,
(b) a metal of the eleventh group of the Periodic Table and
(c) if appropriate, a compound of a metal of the first or second group of the Periodic Table,
these metals being applied to a silica support and the metal of the eleventh group being distributed homogeneously over the cross section of the catalyst particle and more than 80% by weight of the palladium being found in a layer which has a thickness of not more than 0.6 mm and is bounded by the geometric surface of the catalyst particle.

2. The catalyst according to claim 1, wherein the metal of the eleventh group is silver.

3. The catalyst according to claim 1 or 2, wherein palladium is present in an amount of from 0.005 to 1, preferably from 0.02 to 0.6, % by weight, based on the total mass.

4. The catalyst according to any of claims 1 to 3, wherein the metal of the eleventh group is present in an amount of from 0.005 to 1, preferably from 0.05 to 0.6, % by weight and the ratio of metal of the eleventh group to palladium ranges from 0.01 to 100, preferably from 0.5 to 30, particularly preferably from 1.5 to 20.

5. The process for the preparation of a catalyst according to any of claims 1 to 4, wherein first the metal of the eleventh group is applied to the support in such a way that it is homogeneously distributed and then palladium.

6. The process according to claim 5, wherein the application of the metal of the eleventh group is effected before or after the molding of the support, preferably before the molding of the support.

7. The process according to claim 6, wherein the metal of the eleventh group is introduced by kneading into the support material during the edge milling step.

8. The process for the selective hydrogenation of alkynes and dienes in C2-C5⁺-olefin mixtures, preferably in C2- or C3-streams of the olefin preparation by cracking, wherein a catalyst according to any of claims 1 to 4 is used in the hydrogenation.

9. The process according to claim 8, wherein the hydrogenation is to be carried out at from 0 to 250°C and from 0.01 to 50 bar.

## Revendications

1. Catalyseur d'hydrogénation sélective d'alcynes et de diènes en mélanges d'oléfines en C₂-C₅₊, dans lequel ces catalyseurs contiennent :
(a) du palladium;
(b) un métal du onzième groupe du système périodique, et
(c) le cas échéant un composé d'un métal du premier et du deuxième groupe du système périodique,
ces métaux sont appliqués sur un support de dioxyde de silicium et le métal du onzième groupe étant réparti de manière homogène sur la coupe du noyau de catalyseur et se trouvant à plus de 80% en poids du palladium dans une couche de pas plus de 0,6 mm d'épaisseur, qui est délimitée par la surface géométrique de la particule de catalyseur.

2. Catalyseur selon la revendication 1, **caractérisé en ce que** le métal du onzième groupe est l'argent.

3. Catalyseur selon la revendication 1 ou 2, **caractérisé en ce que** le palladium est présent en une quantité de 0,005 à 1% en poids, de préférence de 0,02 à 0,6% en poids, sur base de la masse totale.

4. Catalyseur selon l'une des revendications 1 à 3, **caractérisé en ce que** le métal du onzième groupe est présent dans une quantité de 0,005 à 1% en poids, de préférence de 0,05 à 0,6% en poids, et le rapport du métal du onzième groupe au palladium est de 0,01 à 100, de préférence de 0,5 à 30, le plus préférablement de 1,5 à 20.

5. Procédé de préparation d'un catalyseur selon l'une des revendications 1 à 4, **caractérisé en ce qu'**on applique sur le support d'abord le métal du onzième groupe de telle façon qu'il est réparti de manière homogène, puis on applique le palladium.

6. Procédé selon la revendication 5, **caractérisé en ce que** l'application du métal du onzième groupe s'effectue avant ou après la mise en forme du support, de préférence avant la mise en forme du support.

7. Procédé selon la revendication 6, **caractérisé en ce que** le métal du onzième groupe est incorporé par malaxage dans la masse du support pendant l'étape de broyage-mélange.

8. Procédé d'hydrogénation sélective d'alcynes et de diènes en mélanges d'oléfines en C₂-C₅₊, de préférence en flux en C₂ ou C₃ de la préparation d'oléfines par craquage, **caractérisé en ce qu'**on utilise un catalyseur selon l'une des revendications 1 à 4 lors de l'hydrogénation.

9. Procédé selon la revendication 8, **caractérisé en ce que** l'hydrogénation s'effectue à des températures comprises entre 0 et 250°C et à des pressions de 0,01 à 50 bars.
